Europäisches Patentamt

European Patent Office

Office européen des brevets

(19)

(11) Publication number : **0 062 941**
**B1**

# EUROPEAN PATENT SPECIFICATION

(12)

(45) Date of publication of patent specification :
26.09.84

(51) Int. Cl.³ : **A 61 B  5/10, A 61 B  6/08,**
**G 01 B 11/24**

(21) Application number : 82200361.2

(22) Date of filing : 24.03.82

(54) Contour recording device.

(30) Priority : 08.04.81 NL 8101722

(43) Date of publication of application :
20.10.82 Bulletin 82/42

(45) Publication of the grant of the patent :
26.09.84 Bulletin 84/39

(84) Designated contracting states :
DE FR GB NL SE

(56) References cited :
GB-A-  692 923
GB-A- 1 328 033
NL-A- 7 605 264
US-A- 3 950 096
US-A- 4 242 587
PHYSICS IN MEDICINE AND BIOLOGY, vol. 20, no. 4,
July 1975, pages 627-631, Bristol (GB), S.C. LILLI-
CRAP et al.: "A device for the automatic recording of
patient outlines on the treatment simulator"

(73) Proprietor : **N.V. Philips' Gloeilampenfabrieken**
**Groenewoudseweg 1**
**NL-5621 BA Eindhoven (NL)**

(72) Inventor : **De Vogel, Mathieu Johan**
**c/o INT. OCTROOIBUREAU B.V. Prof. Holstlaan 6**
**NL-5656 AA Eindhoven (NL)**

(74) Representative : **Scheele, Edial François et al**
**INTERNATIONAAL OCTROOIBUREAU B.V. Prof.**
**Holstlaan 6**
**NL-5656 AA Eindhoven (NL)**

# Description

The invention relates to a device for recording object contours in which a contour of the object is marked and the marked contour is sensed and recorded optically.

A device of this kind is known from Physics in Medicine and Biology, vol. 20, n° 4, July 1975, pp. 627-631. Another device of this kind is known from GB 1,328,033. In a device described therein, a light spot is projected onto the object and a probe is automatically adjusted by the detection of reflected light so that the contour can be read during a rotation of the source and the detector about the object. It has been found that this device does not operate satisfactorily in practice. For example, for medical applications the measurement requires too much time and must be separately performed in advance. Differences in reflection from a surface and excessively abrupt angular variations also cause difficulties. The discrete measurement point form of notation required in this device is also considered to be a drawback.

The invention has for its object to mitigate these drawbacks and to provide a device for recording object contours in which a complete contour can be on-line recorded, even in the case of an apparatus in operation. To achieve this, a device of the kind set forth in accordance with the invention is characterized in that the device for the optical recording of a contour comprises a mirror system comprising at least three mirrors arranged about the object to cover the entire contour, a lens associated with each mirror to project sub-images of the marked contour onto a common collecting member to combine the sub-images for the formation of a single image of the marked contour and project it onto the input surface of an image sensing device which, together with the collecting member, is positioned on an isocentric axis.

In an apparatus comprising a device in accordance with the invention, a closed contour can always be displayed on-line on an image display device, without disturbing the measurement or treatment being carried out by the apparatus. Thus, an accurate readily reproducible and fast contour recording can be realized which enables further automatic processing to be simplified.

In a preferred embodiment in accordance with the invention, the optical system comprises, for each different viewpoint a mirror with a lens for forming an angle-corrected sub-image on an optical collecting member in order to form a faithful summed image on, for example, a target of a television camera tube. The optical collecting member may be constructed as a pyramidshaped mirror or as a prism with an entrance side face for each of the mirrors. A lens is arranged preferably, between the collecting member and each of the mirrors, but may alternatively by arranged between the collecting member and the image sensing device so that the sub-images are imaged and combined to form a focussed, continuous and faithful image on the input screen of the image pick-up device.

In a preferred embodiment, the contour to be measured is illuminated by means of light projectors, the number and distribution along a circumference of which may be chosen so that substantially the entire contour will nevertheless be illuminated, even when one of the projector is shielded. A similar distribution may be chosen for the mirrors.

When a computer is included in a device in accordance with the invention, any desired contour can be recorded and used as a reference, for example, to compensate for movements of the object, to prevent a collision between the therapeutic or measuring equipment and the object, to reposition an object, and to monitor any variations in object shape, for example, regression during the irradiation of a patient.

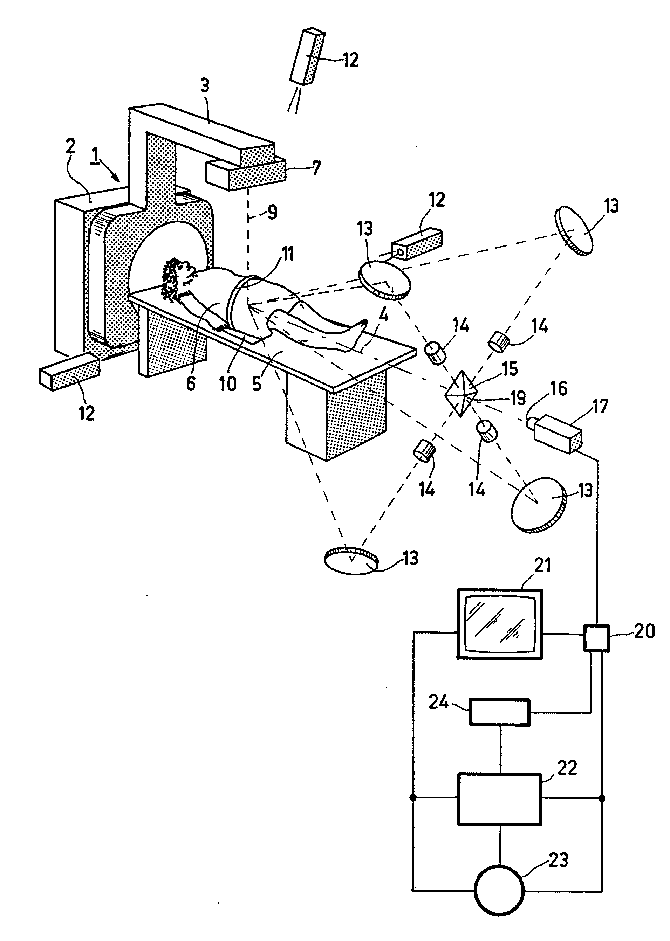

Some preferred embodiments in accordance with the invention will be described in detail hereinafter with reference to the drawing. The single figure of the drawing shows a device in accordance with the invention for measuring body contours for controlling radiation therapy. In a radiation therapy apparatus, for example, a linear accelerator as described in Journal of Applied Medicine, Vol. 5, No. 6, pp. 445-454, the actual linear accelerator comprises a fixed section 1 which is provided with a displacement mechanism for a gantry 2 with an arm 3. The arm 3 is rotatable about a patient 6 arranged on a patient support 5. The patient is irradiated by a beam along a line 9 from a radiation source 7. To achieve this, the table may be provided with an opening 10 in the region of the beam path. By rotating the source about the patient, an anomaly inside the patient can be irradiated while applying a minimum radiation dose to surrounding organs and to the skin of the patient. In order to optimize the irradiation, it is necessary to determine the exact location of the anomaly and to correct for any displacement, and therefore the exact local contour 11 must be known. In order to measure the contour, light sources 12 which together illuminate the entire contour 11, are mounted, for example, on the walls of the room in which the accelerator is arranged. For the sake of clarity, the drawing shows only three light sources ; this number may be sufficient for illuminating a closed contour, but it may be advisable to use a larger number of light sources. In the case of local shielding, for example, by additional equipment or by an operator, the entire contour can then remain illuminated ; the distribution of the light sources over the circumference can also be adapted to the shielding arrangement which is expected to occur most frequently.

In order as far as possible to prevent shielding, notably by operating personnel, it is advantageous to arrange the light sources on the side of the fixed section of the irradiation apparatus. However, this may have disadvantages because of shadows formed by the patient himself; for example, in the case of a contour of the throat, a shadow can readily be formed by the chin. This is avoided by arranging the light sources on the side away from the fixed section of the irradiation apparatus. If both advantages are to be utilized, use can be made of a twin system, so that, for example, a series of mirror systems is mounted on or near the fixed section 1 of the apparatus, and a further series is mounted remote therefrom. Reading can then take place from two directions at mutually equal angles. If a single optical collecting member is used, the optical beam path length must be made the same for both directions in order to obtain an unambiguous image. The device comprises at least three mirrors 13, and the embodiment shown comprises four mirrors. These mirrors may be secured to the walls of the room in such an arrangement that the entire contour can be seen from the combination of mirrors. The positions of the mirrors need not correspond to those of the light sources, but such correspondence need not be excluded. Each of the mirrors has associated with it a lens 14 which images that part of the contour line which is reflected by the mirror onto the entrance target 16 of a television camera 17 *via* an optical collecting member 15. The optical axis of the television camera and an optical centre 19 of the collecting member coincide with an axis 4 about which the source rotates and which is usually referred to as the isocentric axis. The television camera is preferably connected, *via* a switching device 20, to a television monitor 21 for displaying the contour. A contour thus sensed and measured can be stored in digital form in the memory of a computer 22 or in analog form on a magnetic tape or on a disk 23. Once a contour of an object has been sensed, measured and stored, it can be used as a reference contour and a simple indicator 24 then suffices to signal any discrepancies occurring. If desired, the reference contour and a currently measured contour can be displayed together on the monitor. This enables a direct visual observation to be made of any discrepancy in the latest contour and, for example, an undesired shift of, for example, an arm of the patient can be corrected immediately. It is then also simple to add an automatic switch-off device for the irradiation apparatus which responds to a predetermined discrepancy in the contour. From a sensed and measured contour a circumscribed circle can be calculated whose coordinates can be used for controlling an anti-collision device included in the apparatus, which maintains a minimum clearance between the patient and the relevant parts of the patient support or the equipment used during therapy, for example, a detector or radiation shield. An identified and recorded contour can also be used for repositioning the patient, for example, for repeating a course of radiation therapy.

For the present embodiment it has been assumed that the light sources and the mirrors each occupy a fixed position. If the mirrors are secured to a rotating part, for example, the arm 3, it is desirable that the lenses, the collecting member and the camera should also be secured thereto. In that case, the light sources may be arranged to be stationary or to be rotatable, as desired. In the case of an arrangement involving camera rotation, it may be advantageous to apply image rotation to the monitor display, so that the orientation of the picture on the screen remains fixed. In order to reduce the disturbing effect of ambient light on the measurement, use can be made of light sources generating light having a specific wavelength to which the measuring device is adapted. The detection device of the described embodiment comprises a television camera. Instead of a television camera, use can be made of an alternative image pick-up device which may be cheaper because its resolution need not be very high. A suitable detector in this respect could be a self-scanning semiconductor detector.

The invention has been described with reference to a medical therapeutic apparatus, notably a linear accelerator. It will be apparent that the invention has a much wider field of application, for example, other medical equipment where the separate measurement of a contour is important, for example, in scanners in cobalt irradiation apparatus, neutron irradiation apparatus and the like. The invention can also be successfully used for non-medical applications, such as for the measurement of objects or workpieces to be treated and for determining shape variations caused in objects by physical effects. A device in accordance with the invention can also be used for the fast and accurate testing of workpieces with respect to given dimensions, and the profiles of moulds used for moulding, can be simply determined and recorded. In the foregoing, a contour marked by light sources has been assumed. Even though this is a convenient method, use can alternatively be made of a contour marked in a different manner, for example, by the application of a dye, a fluorescent substance or a series of luminescent devices. In that case the scanning section may still have the described construction. Therefore, the invention can also be used for sensing and measuring contour marks which already form part of an object such as a workpiece or a mould.

### Claims

1. A device for recording object contours in which a contour of an object is marked and the marked contour is sensed and recorded optically, characterized in that an optical system of the device comprises a mirror system comprising at least three mirrors (13) arranged about the object to cover the entire contour, a lens (14) associated with each mirror to project sub-images of the marked contour onto a common collecting member (15) to combine the sub-images for the formation of a single image of the marked contour and project it onto the input surface of an image sensing device (17) which, together with the collecting member, is positioned on an isocentric axis (4).

2. A device as claimed in Claim 1, characterized in that the lenses (4) form an angle-corrected faithful sub-image of a contour portion on the optical collecting member which forms from the sub-images a focussed, faithful image of the contour on the image-forming device.

3. A device as claimed in Claim 2, characterized in that the mirror system is of duplicate construction with mutually equal reading angles and mutually equal optical beam path lengths to a common optical collecting member.

4. A device as claimed in Claim 1, 2 or 3, characterized in that the image-sensing device comprising a television camera to which a monitor is adapted.

5. A device as claimed in Claim 4, characterized in that a video memory is associated therewith.

6. A device as claimed in Claim 4 or 5, characterized in that an analog-to-digital converter and a computer are associated therewith for the recording and comparison of contours.

7. A medical irradiation apparatus comprising a device for recording object contours as claimed in any one of the preceding Claims.

8. An apparatus for a treatment of work pieces, comprising a device for recording object contours as claimed in any one of Claims 1 to 6.

## Ansprüche

1. Gerät zum Aufzeichnen von Umrissen, wobei der Umriss eines Objekts markiert ist und der markierte Umriss optisch abgetastet und aufgezeichnet wird, dadurch gekennzeichnet, dass ein optisches System des Geräts ein Spiegelsystem mit zumindest drei um das Objekt herum angeordneten Spiegeln (13) zum Erfassen des ganzen Umrisses und eine Linse (14) enthält, die jedem Spiegel zugeordnet ist und Teilbilder des markierten Umrisses auf ein gemeinsames Sammelelement (15) zum Kombinieren der Teilbilder zur Bildung eines einzigen Bildes des markierten Umrisses und zum Projizieren dieses Bildes auf die Eintrittsfläche eines Bildabtastgeräts (17) projiziert, das zusammen mit dem Sammelelement auf einer isozentrischen Achse (4) angeordnet ist.

2. Gerät nach Anspruch 1, dadurch gekennzeichnet, dass die Linsen (4) ein winkelkorrigiertes getreues Teilbild eines Umrissteils auf dem optischen Sammelelement bilden, das aus den Teilbildern ein fokussiertes, getreues Bild des Umrisses auf dem Bildformungsgerät formt.

3. Gerät nach Anspruch 2, dadurch gekennzeichnet, dass das Spiegelsystem in zweifacher Ausführung mit gleichen Lesewinkeln und gleichen optischen Bündelweglängen zu einem gemeinsamen optischen Sammelelement aufgebaut ist.

4. Gerät nach Anspruch 1, 2 oder 3, dadurch gekennzeichnet, dass das Bildabtastgerät eine Fernsehkamera enthält, an die ein Minitor angeschlossen ist.

5. Gerät nach Anspruch 4, dadurch gekennzeichnet, dass ein Videospeicher zugeordnet ist.

6. Gerät nach Anspruch 4 oder 5, dadurch gekennzeichnet, dass ein Analog/Digital-Wandler und ein Computer zum Aufzeichnen und Vergleichen der Umrisse angeschlossen sind.

7. Medizinisches Strahlungsgerät mit einer Einrichtung zum Aufzeichnen von Umrissen nach einem oder mehreren der vorangehenden Ansprüche.

8. Apparat für die Werkstückbehandlung, mit einem Gerät zum Aufzeichnen von Umrissen nach einem der Ansprüche 1 bis 6.

## Revendications

1. Dispositif pour enregistrer des contours d'objet dans lequel un contour d'un objet est marqué et le contour marqué est détecté et enregistré par voie optique, caractérisé en ce qu'un système optique comprend un système de miroirs comprenant au moins trois miroirs (13) disposés autour de l'objet afin de couvrir tout son contour, un objectif (14) associé à chaque miroir pour projeter les sous-images du contour marqué sur un élément collecteur commun (15) afin de combiner les sous-images en vue de former une seule image du contour marqué et la projeter sur la surface d'entrée d'un dispositif détecteur d'image (17) qui, en même temps que l'élément collecteur, est positionné sur un axe isocentrique (4).

2. Dispositif suivant la revendication 1, caractérisé en ce que les objectifs (4) forment une sous-image fidèle angulairement corrigée d'une partie du contour sur l'élément collecteur optique qui forme à partir des sous-images une image fidèle et focalisée du contour sur le dispositif formateur d'image.

3. Dispositif suivant la revendication 2, caractérisé en ce que le système à miroirs est de construction double et présente des angles de lecture réciproquement égaux et des longueurs de trajets de faisceaux optiques réciproquement égales vers un élément collecteur optique commun.

4. Dispositif suivant la revendication 1, 2 ou 3, caractérisé en ce que le dispositif détecteur d'image comprend une caméra de télévision à laquelle est adapté un moniteur.

5. Dispositif suivant la revendication 4, caractérisé en ce qu'une mémoire vidéo y est associée.

6. Dispositif suivant la revendication 4 ou 5, caractérisé en ce qu'un convertisseur analogique-numérique et un ordinateur y sont associés pour l'enregistrement et la comparaison de contours.

7. Appareil d'irradiation médical comprenant un dispositif pour enregistrer des contours d'objet suivant l'une quelconque des revendications précédentes.

8. Appareil pour le traitement d'ouvrage, comprenant un dispositif pour enregistrer des contours d'objet suivant l'une quelconque des revendications 1 à 6.